# EUROPEAN PATENT SPECIFICATION

(11) **EP 2 245 007 B1**
(45) Date of publication and mention of the grant of the patent: **19.08.2020**
(21) Application number: 09714810.0
(22) Date of filing: 26.02.2009
(51) Int. Cl.: C07D 207/16, A61K 31/401, A61P 9/04, A61P 9/10, A61P 9/12

(54) **Process for preparing a crystalline form of zofenopril calcium**
Verfahren zur Herstellung von einer kristallinen Form von Zofenopril Calcium
Procédé de préparation d'une forme cristalline du zofenopril calcium

(30) Priority: 27.02.2008 IN KA03542008
(43) Date of publication of application: 03.11.2010
(73) Proprietor: Generics [UK] Limited, Hatfield, Hertfordshire AL10 9UL (GB)
(72) Inventor: GORE, Vinayak, Maharashtra 410208 (IN); VIJAYAKAR, Priyesh, Maharashtra 410208 (IN); PEHERE, Ashok, Maharashtra 410208 (IN)
(74) Representative: FRKelly
(86) International application number: PCT/GB2009/050199
(87) International publication number: WO 2009/106894

(56) References cited:
- WO-A-2007/138352
- WO-A-2009/022168
- US-A- 4 316 906
- US-B2- 6 515 012

## Description

### Field of the invention

The present invention is directed to a process for preparing a polymorphic form of zofenopril calcium. The present disclosure also relates to a novel crystalline form of zofenopril calcium, to processes for their preparation and their use in pharmaceutical compositions.

### Background of the invention

Zofenopril calcium of formula (I), chemically named (4S)-1-[(2S)-3-(benzoylthio)-2-methylpropionyl]-4-(phenylthio)-L-proline calcium salt, is a non-peptidic orally active sulphydryl ACE inhibitor with a long-lasting action and it is currently marketed for the treatment of hypertension.

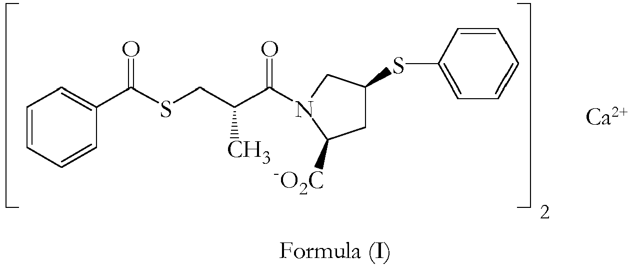

The manufacturing process for many pharmaceuticals is hindered by the fact that the organic compound which is the active ingredient has handling difficulties during the manufacturing process and may impart undesirable properties to the final drug or dosage form. In addition it can be difficult to control the polymorphic form of the active pharmaceutical ingredient throughout the manufacturing process.

For pharmaceuticals in which the active ingredient can exist in more than one polymorphic form, it is particularly important to ensure that the manufacturing process for the active ingredient affords a single polymorph with a consistent level of polymorphic purity. If the process leads to a polymorph with varying degrees of polymorphic purity and/or where the process does not control polymorphic interconversion, serious problems in dissolution and/or bioavailability can result in the finished pharmaceutical composition comprising the active ingredient.

Zofenopril calcium polymorphs are disclosed in patents US 6,515,012 and US 4,316,906. The method for the preparation of zofenopril calcium as disclosed in US 4,316,906 is described in US 6,515,012 as comprising steps as follows:
(a) condensation between cis-4-(phenylthio)-L-proline and (D)-3-(benzoylthio)-2-methylpropionyl chloride in aqueous solution keeping the pH at values of 8-8.5 by addition of 5N sodium hydroxide, subsequent acidification with HCl, extraction with isobutyl acetate and concentration of the extracts, washing with saline solution, to give (4S)-1-[(2S)-3-(benzoylthio)-2-methylpropinoyl]-4-(phenylthio)-L-proline;
(b) treatment of the resinous material from the previous step in isopropanol solution with potassium 2-ethyl-hexanoate to obtain the corresponding potassium salt;
(c) dissolution of the potassium salt in water to a 57% concentration and very slow addition, with simultaneous seeding, of a slight excess of a 2N calcium chloride aqueous solution to precipitate the desired calcium salt, washing the resulting product thoroughly with water, drying under vacuum at a comparatively high temperature to give the desired calcium salt as dry powder with a melting point of about 250°C;
(d) alternatively, (4S)-1-[(2S)-3-(benzoylthio)-2-methylpropinoyl]-4-(phenylthio)-L-proline is dissolved in ethanol and treated with the same volume of an aqueous suspension containing one equivalent of CaO; after removing ethanol and subsequently washing with ether, the aqueous suspension is freeze-dried to obtain the calcium salt with a melting point of 235-237°C.

According to US 6,515,012, the synthesis described in US 4,316,906 (cited above at points a, b and c) mainly yields polymorph form A, but also polymorph form B in very variable percentages and never below 20%. Moreover, the alternative synthesis described (cited at point d) affords a partially amorphous product with very variable characteristics in which form A, when present, is in concentrations much lower than those obtained in the preceding process.

US 6,515,012 and US 6,521,760 disclose a process for the preparation of substantially pure polymorph form A from zofenopril calcium, comprising the following steps:
(a) reaction of S(-)-3-(benzoylthio)-2-methyl-propanoic acid chloride and cis-4-(phenylthio)-L-proline in water at a pH ranging from 9.0-9.5 and recovery of zofenopril in its acidic form;
(b) salification of acid zofenopril with a potassium salt in alcoholic solution and recovery of the resulting potassium salt;
(c) conversion of the potassium salt to the calcium salt by addition of an aqueous solution of zofenopril potassium salt to a calcium chloride aqueous solution at 70-90°C with simultaneous seeding to promote the precipitation of polymorph form A.

However, the synthesis disclosed in the aforesaid US patents for the preparation of polymorph form A has the disadvantage that the reaction is carried out at a relatively high temperature (80-85°C) at which interconversion of the polymorphs is possible. Consequently, although substantially pure form A can be obtained from the above process, it is not very reliable and the possibility of traces of form B cannot be completely eliminated.

The aforesaid US patents also disclose a process for the preparation of polymorph form B, comprising the following steps:
(a) A solution of zofenopril potassium salt (0.27M) is sprayed in lukewarm water (55°C), while adding a calcium chloride solution, the solution being such that the total amount of drug and calcium chloride are equimolar.
(b) The resulting suspension containing the slurry product is heated at 85°C for 12-14 hours to obtain complete conversion to form B.
(c) After cooling at about 25°C, the product is filtered, washed with water until it is substantially free from chloride ions, and then dried under vacuum.

WO 2007/003963 discloses a process for the preparation of substantially pure polymorph form C (monohydrate form) of zofenopril calcium, comprising the following steps:
(a) reaction of S(-)-3-(benzoylthio)-2-methyl-propanoic acid chloride and cis-4-(phenylthio)-L-proline in water at a pH ranging from 9.0-9.5 and recovery of zofenopril in its acidic form;
(b) salification of acid zofenopril with a potassium salt in alcoholic solution and recovery of the resulting potassium salt;
(c) conversion of the potassium salt to the calcium salt by addition of an aqueous solution of calcium chloride dihydrate to an aqueous solution of zofenopril potassium salt at 50-55°C.

The polymorph form C described in the aforesaid WO 2007/003963 patent application has the advantage that it is prepared at milder conditions than the experimental conditions reported for the polymorphs A and B. In addition, it was found to be purer with respect to contamination by other forms (polymorph forms A and B) as indicated by XRPD data, and generally more stable to polymorphic interconversion.

However, although the properties of form C and the processes to prepare it are generally better and more convenient that those described for previous polymorphic forms, it has been observed that under certain conditions during dosage form preparation, such as micronization or wet granulation, the form C product can exhibit very slight changes in polymorphic purity. Although this difference was only to a very small extent, it could lead to variation in dissolution profile and subsequent problems in the pharmaceutical composition development.

Although formulation development may be able to circumvent the potential problems with form C, it was considered that an alternative polymorphic form with improved properties over all the known polymorphic forms may make development and manufacture more convenient and efficient and could also lead to improved pharmaceutical compositions.

WO 2009/022168 relates to a process for the preparation of the new crystalline form D of zofenopril calcium, its use in pharmaceutical compositions and the use of the new crystalline form and compositions in the treatment of hypertension and various other diseases.

### Object of the invention

Therefore an object of the invention is to provide a new process for preparing a polymorphic form of zofenopril calcium, which is convenient to manufacture and has improved properties suitable for formulation development and a marketed pharmaceutical composition.

### Summary of the invention

The present inventors have surprisingly developed a new process for preparing a polymorphic form of zofenopril calcium with improved properties which circumvents the problems associated with the polymorphic forms reported in the prior art as described above.

The new polymorphic form of zofenopril calcium has been designated as form E and is a crystalline anhydrous form of zofenopril calcium.

Form E of zofenopril calcium is more polymorphically pure and stable than the polymorphic forms reported in the prior art. In particular, form E is stable to stress conditions during dosage form preparation (see example 11).

The present inventors have also developed convenient processes for the preparation of the novel forms E and F under mild conditions.

The invention provides a process for preparing a polymorphic form of zofenopril calcium, comprising the steps of:
(a) dissolving a salt of zofenopril in a water miscible organic solvent;
(b) mixing the solution obtained in step (a) with an aqueous solution of a calcium salt; and
(c) isolating the polymorphic form of zofenopril calcium;
wherein the polymorphic form is characterised by an XRPD spectrum comprising at least six of the following 2θ peaks: 4.2, 4.8, 9.6, 17.5, 18.0, 19.3, 19.9, 20.6 and 24.4 ± 0.2 degrees 2θ;
with the proviso that the salt of zofenopril in step (a) is not an amine salt and wherein steps (a) and (b) are carried out at a temperature of up to 30°C

The polymorphic form may be characterised by a differential scanning calorimetry (DSC) with an endothermic peak at about 255°C.

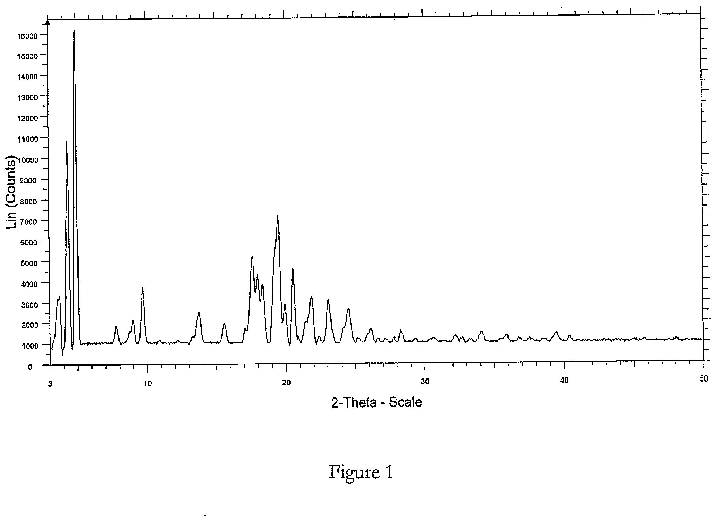

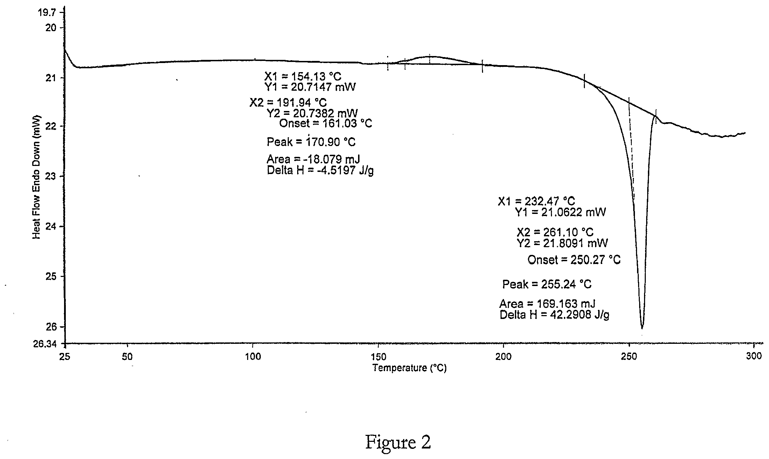

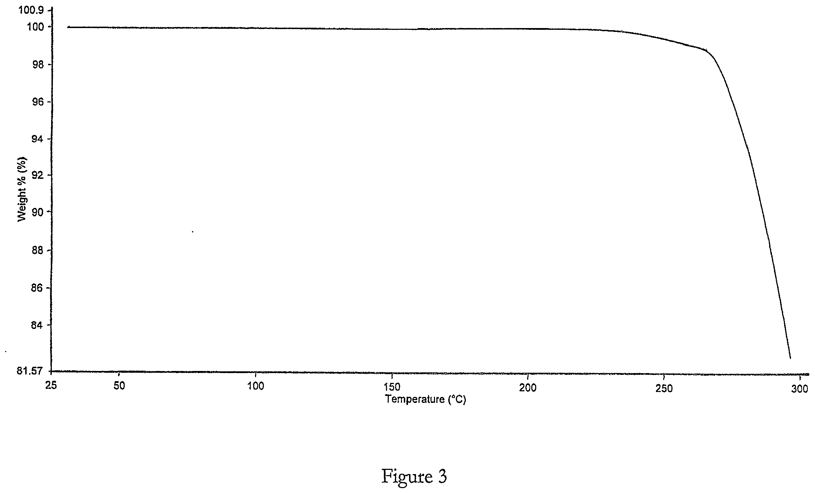

The polymorphic form may be characterised by:
(i) an XRPD spectrum substantially as shown in Figure 1; and/or
(ii) a DSC trace substantially as shown in Figure 2; and/or
(iii) a TGA trace substantially as shown in Figure 3.

The polymorphic form typically comprises less than 10%, less than 5%, less than 1%, less than 0.5%, or less than 0.1% of zofenopril calcium in other polymorphic or amorphous forms.

In one embodiment the water miscible organic solvent is an alcohol, an ether, a cyclic ether, a ketone or an amide.

Typically the water miscible organic solvent is:
(i) an alcohol; and/or
(ii) a straight-chain, branched or cyclic C₁ to C₆ alcohol; and/or
(iii) selected from methanol, ethanol, propanol, isopropanol, n-butanol or mixtures thereof.

In one embodiment the water miscible organic solvent is:
(i) a cyclic ether; and/or
(ii) selected from tetrahydrofuran and dioxane or mixtures thereof.

In one embodiment the water miscible organic solvent is:
(i) an amide; and/or
(ii) selected from N,N-dimethylformamide, formamide and N,N-dimethylacetamide or mixtures thereof.

The the zofenopril salt in step (a) may be a metal salt.

Typically the metal salt is a potassium, sodium, lithium, calcium, magnesium or aluminium salt.

In one embodiment the metal salt is a potassium salt.

Typically the calcium salt in step (b) is selected from the fluoride, chloride, bromide, iodide, oxide, hydroxide, carbonate, nitrate, sulfate or acetate salts.

In one embodient the calcium salt is calcium chloride.

Typically the is carried out at a temperature of from 20 to 30°C.

Therefore, in a first aspect of the present invention there is provided zofenopril calcium form E characterised by an XRPD spectrum comprising at least four of the following 2θ peaks (preferably at least five, six, seven, eight, or all nine peaks): 4.2, 4.8, 9.6, 17.5, 18.0, 19.3, 19.9, 20.6 and 24.4 ± 0.2 degrees 2θ. Preferably the zofenopril calcium form E has an XRPD spectrum substantially as shown in Figure 1.

Form E may be further characterized by a differential scanning calorimetry (DSC) with an endothermic peak at about 255°C, preferably at about 255.2°C. Preferably the zofenopril calcium form E has a DSC trace substantially as shown in Figure 2.

Preferably the zofenopril calcium form E has a TGA trace substantially as shown in Figure 3.

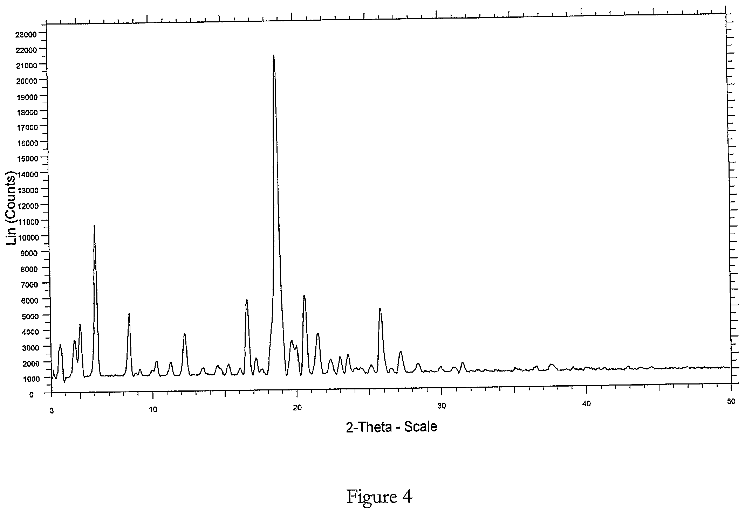

Zofenopril calcium form F disclosed herein but not claimed is characterised by an XRPD spectrum comprising at least four of the following 2θ peaks (preferably at least five, six, seven, eight, nine, ten, or all eleven peaks): 4.6, 6.0, 8.3, 10.2, 14.5, 16.5, 17.1, 18.2, 19.9, 21.4 and 23.5 ± 0.2 degrees 2θ. Preferably the zofenopril calcium form F has an XRPD spectrum substantially as shown in Figure 4.

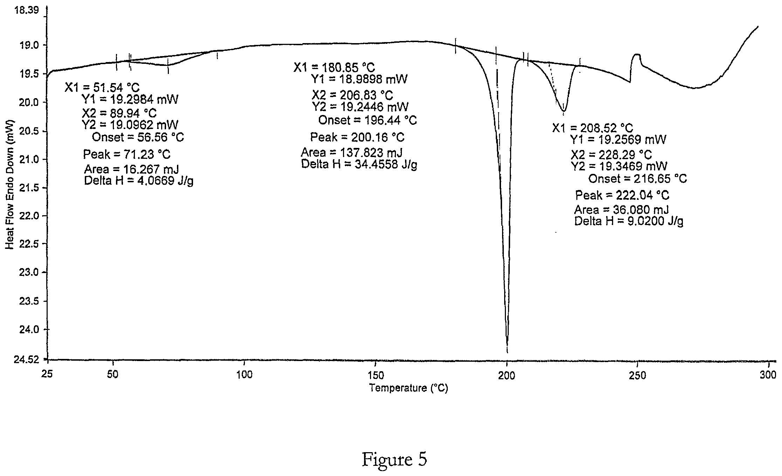

Form F may be further characterized by a differential scanning calorimetry (DSC) with an endothermic peak at about 200°C, preferably at about 200.2°C. Preferably the zofenopril calcium form F has a DSC trace substantially as shown in Figure 5.

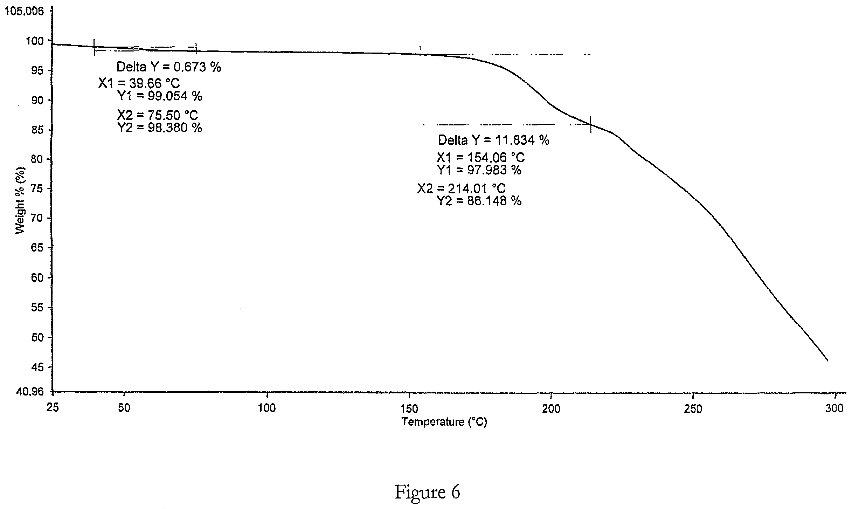

Preferably the zofenopril calcium form F has a TGA trace substantially as shown in Figure 6.

According to the present invention there is provided a process for preparing zofenopril calcium form E, comprising the steps of:
(a) dissolving a salt of zofenopril in a water miscible organic solvent;
(b) mixing the solution obtained in step (a) with an aqueous solution of a calcium salt; and
(c) isolating the zofenopril calcium form E; with the proviso that the salt of zofenopril in step (a) is not an amine salt.

Preferably, the water miscible organic solvent is an alcohol, an ether, a cyclic ether, a ketone or an amide.

Preferably, the water miscible organic solvent is an alcohol, such as a straight-chain, branched or cyclic C₁ to C₆ alcohol. More preferably, the alcohol is selected from methanol, ethanol, propanol, isopropanol, n-butanol or mixtures thereof.

Alternatively, the water miscible organic solvent is a cyclic ether, preferably selected from tetrahydrofuran and dioxane or mixtures thereof.

Alternatively, the water miscible organic solvent is an amide, preferably selected from N,N-dimethylformamide, formamide and N,N-dimethylacetamide or mixtures thereof.

Preferably, the zofenopril salt in step (a) is a metal salt, which is preferably selected from a potassium, sodium, lithium, calcium, magnesium or aluminium salt. Most preferably, the metal salt is a potassium salt.

Preferably, the calcium salt in step (b) is selected from the fluoride, chloride, bromide, iodide, oxide, hydroxide, carbonate, nitrate, sulfate or acetate salts. Most preferably, the calcium salt is calcium chloride.

A pharmaceutical composition comprising zofenopril calcium form E may be used to treat or prevent a disorder wherein inhibiting an angiotensin converting enzyme (ACE) is beneficial. The disorder may be selected from hypertension, cardiac decompensation, myocardial infarction, acute myocardial infarction, heart failure or chronic heart failure.

Zofenopril calcium form E preferably comprises less than 10%, preferably less than 5%, preferably less than 1%, preferably less than 0.5%, and most preferably less than 0.1% of zofenopril calcium in other polymorphic or amorphous forms (as measured by XRPD or DSC). Preferably the zofenopril calcium form E has a chemical purity of 95%, 97%, 98%, 99%, 99.5%, 99.9% or more (as measured by HPLC).

Zofenopril calcium form E may be used for the manufacture of a medicament for the treatment or prevention of a disorder wherein inhibiting an angiotensin converting enzyme (ACE) is beneficial. Preferably, the disorder is selected from hypertension, cardiac decompensation, myocardial infarction, acute myocardial infarction, heart failure or chronic heart failure.

The invention may be used in a method of treating or preventing a disorder wherein inhibiting an angiotensin converting enzyme (ACE) is beneficial, the method comprising administering to a patient in need thereof a therapeutically or prophylactically effective amount of zofenopril calcium form E. The disorder may be selected from hypertension, cardiac decompensation, myocardial infarction, acute myocardial infarction, heart failure or chronic heart failure. Preferably, the patient is a mammal, preferably a human.

### Brief description of the accompanying figures

Figure 1: X-ray powder diffraction (XRPD) of zofenopril calcium form E.
Figure 2: Differential scanning calorimetry (DSC) of zofenopril calcium form E.
Figure 3: Thermo-gravimetric analysis (TGA) of zofenopril calcium form E.
Figure 4: X-ray powder diffraction (XRPD) of zofenopril calcium form F.
Figure 5: Differential scanning calorimetry (DSC) of zofenopril calcium form F.
Figure 6: Thermo-gravimetric analysis (TGA) of zofenopril calcium form F.

### Detailed description of the invention

The terms 'crystalline form', 'polymorphic form' and the like are used interchangeably herein.

As outlined above, the present invention provides a new crystalline form of zofenopril calcium, form E, which is non-hygroscopic, polymorphically pure and stable and has beneficial properties which avoid the problems associated with prior art forms.

The differences in XRPD data between the novel forms E and F and prior art forms of zofenopril calcium are illustrated in Table 1.

**Table 1: XRPD comparison [2θ values]**

| **Polymorph A (**WO00/07984**)** | **Polymorph B (**WO00/07984**)** | **Polymorph C (**WO07/03963**)** | **Polymorph E** | **Polymorph F** |
|---|---|---|---|---|
| | | | 4.2 | |
| 4.3 | | | | |
| | | | | 4.6 |
| | 4.8 | | 4.8 | |
| | | 4.9 | | 4.9 |
| | | | | 6.0 |
| 7.4 | | | | |
| | | 8.1 | | |
| | | | | 8.3 |
| 8.7 | | | | |
| | | 9.1 | | |
| | | | 9.6 | |
| | | 9.9 | | |
| 10.1 | | | | |
| | | | | 10.2 |
| 10.8 | | | | |
| 11.7 | | | | |
| | | | | 12.2 |
| 13.0 | | | | |
| | | | 13.7 | |
| | | 14.5 | | 14.5 |
| 14.8 | | | | |
| | 15.6 | | | |
| 16.0 | | 16.0 | | |
| | | | | 16.5 |
| 17.1 | | | | 17.1 |
| | 17.5 | 17.5 | 17.5 | |
| | | | 18.0 | |
| 18.2 | | | | 18.2 |
| | 18.5 | 18.5 | | |
| | | | | 18.7 |
| 19.0 | | 19.0 | | |
| | | | 19.3 | |
| | 19.4 | | | |
| | | | 19.9 | 19.9 |
| 20.0 | | 20.0 | | |
| | 20.5 | 20.5 | | 20.5 |
| | | | 20.6 | |
| | 21.4 | | | 21.4 |
| | | 21.5 | | |
| 21.7 | | | | |
| | 21.8 | | 21.8 | |
| | | 22.3 | | |
| | | | | 22.9 |
| | | | 23.0 | |
| | 23.1 | | | |
| 23.5 | | | | 23.5 |
| | | 23.9 | | |
| | | | 24.4 | |
| | | 24.5 | | |
| 24.6 | 24.6 | | | |
| | | | | 25.7 |
| | | | | 27.1 |

In addition, convenient processes for the preparation of forms E and F has been provided by the present invention. These processes use mild conditions and low temperatures, thus minimizing the occurrence of polymorphic interconversion and producing form E with very high polymorphic purity.

Preferred embodiments of the processes according to the present invention are described below.

A preferred process for the preparation of zofenopril calcium form E comprises: adding an aqueous solution of calcium chloride dihydrate to a clear water miscible organic solvent solution of zofenopril potassium salt at 25-30°C. The resulting suspension is stirred for 4 hours. The suspension is filtered at 25-30°C and the product is washed with water until substantially free from chloride ions. The water miscible organic solvents can include alcohols (such as methanol, ethanol, propanol, isopropanol, or n-butanol), cyclic ethers (such as tetrahydrofuran or dioxane) and amides (such as N,N-dimethylformamide or N,N-dimethylacetamide). Preferably, the wet solids are dried under reduced pressure at 65°C until the moisture content falls below 0.5%.

The XRPD pattern of the form E product thus obtained is different from the reported polymorph A, polymorph B and polymorph C of zofenopril calcium as represented in Table 1.

Polymorph E is also obtained in the case of reverse addition in the first step, when a clear solution of zofenopril potassium salt is added to a clear aqueous solution of calcium chloride dihydrate.

The temperature employed in the process for the preparation of the novel crystalline form E of zofenopril calcium is preferably from 20-30°C, and more preferably from 25-30°C.

In the processes according to the present invention, the reaction mixture is maintained at a temperature of up to 30°C for 2 to 30 hours before filtering the suspension. Alternatively, the reaction mixture is preferably maintained at a temperature of from 25 to 30°C for 3 to 30 hours before filtering the suspension.

Preferably, the two salt solutions are mixed at a temperature of up to 30°C.

Preferably, the zofenopril calcium isolated is washed with water, preferably until substantially free from chloride ions.

Preferably, the zofenopril calcium isolated is dried, preferably under reduced pressure. Preferably, the zofenopril calcium is dried at a temperature of up to 70°C, preferably up to 60°C. Preferably, the zofenopril calcium is dried until the moisture content falls below about 1%, preferably below about 0.5%.

Preferably, the temperature throughout substantially the whole process according to the present invention is kept at 70°C or less, preferably at 60°C or less. For the purposes of the present invention, the temperature is kept at 70°C or less 'throughout substantially the whole process', even if the temperature occasionally rises above 70°C, provided this rise in temperature does not influence the polymorphic form or polymorphic purity of the zofenopril calcium obtained.

The major advantage of this invention is milder experimental temperature conditions of the process to obtain the novel polymorphs and the polymorphic purity and stability of the form E and form F. The polymorphic forms of the present invention also allow zofenopril calcium to be easily purified and obtained in very high chemical purity.

The pharmaceutical composition described herein can be a solution or a suspension, but is preferably a solid oral dosage form. Preferred oral dosage forms in accordance with the invention include tablets, capsules and the like which, optionally, may be coated if desired. Tablets can be prepared by conventional techniques, including direct compression, wet granulation and dry granulation. Capsules are generally formed from a gelatine material and can include a conventionally prepared granulate of excipients in accordance with the invention.

The pharmaceutical composition described herein typically comprises one or more conventional pharmaceutically acceptable excipient(s) selected from the group comprising a filler, a binder, a disintegrant, a lubricant, and optionally further comprises at least one excipient selected from colouring agents, adsorbents, surfactants, film formers and plasticizers.

If the solid pharmaceutical formulation is in the form of coated tablets, the coating may be prepared from at least one film-former such as hydroxypropyl methyl cellulose, hydroxypropyl cellulose or methacrylate polymers which optionally may contain at least one plasticizer such as polyethylene glycols, dibutyl sebacate, triethyl citrate, and other pharmaceutical auxiliary substances conventional for film coatings, such as pigments and fillers.

Preferably, the pharmaceutical compositions described herein are for use in treating or preventing disorders where inhibiting an angiotensin converting enzyme (ACE) is beneficial. Such disorders include, but are not limited to, hypertension, cardiac decompensation, myocardial infarction, acute myocardial infarction, heart failure and chronic heart failure.

Preferably the pharmaceutical compositions described herein are in unit dosage form comprising zofenopril calcium in an amount of from 1 mg to 500 mg. The unit dosage form can be administered once, twice, three times, four times or more per day. Preferably the amount of zofenopril calcium administered is from 0.1 mg to 100 mg per kg per day.

The details of the invention, its objects and advantages are illustrated below in greater detail by non-limiting examples.

### Examples

### Form E

The preparation of crystalline form E of zofenopril calcium is illustrated in examples 1 to 4. The products obtained exhibited identical data as depicted in Figures 1, 2 and 3.

### Example 1

To a clear alcoholic (methanol, ethanol, propanol, isopropanol, n-butanol) solution of zofenopril potassium salt, an aqueous solution of calcium chloride dihydrate was added at 25-30°C and the resulting suspension was stirred for 4 hours at 25-30°C. Then this suspension was filtered at 25-30°C and the product was washed with water until free from chloride ions. The wet solids were dried under reduced pressure at 60°C until the moisture content fell below 0.5%.
Polymorphic purity >99.9% (as measured by XRPD and DSC)
Chemical purity >99.6% (as measured by HPLC)

### Example 2

To a clear aqueous solution of calcium chloride dihydrate, an alcoholic (methanol, ethanol, propanol, isopropanol, n-butanol) solution of zofenopril potassium salt was added at 25-30°C. The temperature of the reaction mixture was maintained for 4 hours at 25-30°C. Then this suspension was filtered at 25°C and the product was washed with water until free from chloride ions. The wet solids were dried under reduced pressure at 60°C until the moisture content fell below 0.5%.
Polymorphic purity >99.9% (as measured by XRPD and DSC)
Chemical purity >99.3% (as measured by HPLC)

### Comparative Example 3

To a clear alcoholic (methanol, ethanol, propanol, isopropanol, n-butanol) solution of zofenopril potassium salt, an aqueous solution of calcium chloride dihydrate was added at 50-55°C and the resulting suspension was stirred for 4 hours at 50-55°C. Then this suspension was filtered at 50-55°C and the product was washed with water until free from chloride ions. The wet solids were dried under reduced pressure at 60°C until the moisture content fell below 0.5%.
Polymorphic purity >99.9% (as measured by XRPD and DSC)
Chemical purity >99.6% (as measured by HPLC)

### Comparative Example 4

To a clear aqueous solution of calcium chloride dihydrate, an alcoholic (methanol, ethanol, propanol, isopropanol, n-butanol) solution of zofenopril potassium salt was added at 50-55°C. The temperature of the reaction mixture was maintained for 4 hours at 50-55°C. Then this suspension was filtered at 50-55°C and the product was washed with water until free from chloride ions. The wet solids were dried under reduced pressure at 60°C until the moisture content fell below 0.5%.
Polymorphic purity >99.9% (as measured by XRPD and DSC)
Chemical purity >99.3% (as measured by HPLC)

The same crystalline form of zofenopril calcium (form E) was obtained using cyclic ethers (tetrahydrofuran, dioxane) and amides (N,N-dimethylformamide, formamide, N,N-dimethylacetamide) as solvent.

### Reference Form F

The preparation of crystalline form F of zofenopril calcium is illustrated in examples 5 to 10. The products obtained exhibited identical data as depicted in Figures 4, 5 and 6.

### Reference Example 5

To a clear alcoholic (methanol, ethanol, propanol, isopropanol, n-butanol) solution of zofenopril dicyclohexylamine salt, an aqueous solution of calcium chloride dihydrate was added at 25-30°C and the resulting suspension was stirred for 3 hours 25-30°C (alcohol : water ratio 1:1). Then this suspension was filtered at 25-30°C and the product was washed with water until free from chloride ions. The wet solids were dried under reduced pressure at 55°C until the moisture content fell below 0.5%.
Polymorphic purity >99.9% (as measured by XRPD and DSC)
Chemical purity >99.5% (as measured by HPLC)

### Reference Example 6

To a clear alcoholic (methanol, ethanol, propanol, isopropanol, n-butanol) solution of zofenopril dicyclohexylamine salt, an aqueous solution of calcium chloride dihydrate was added at 25-30°C and the resulting suspension was stirred for 3 hours 25-30°C (alcohol : water ratio 1:3). Then this suspension was filtered at 25-30°C and the product was washed with water until free from chloride ions. The wet solids were dried under reduced pressure at 55°C until the moisture content fell below 0.5%.
Polymorphic purity >99.9% (as measured by XRPD and DSC)
Chemical purity >99.5% (as measured by HPLC)

### Reference Example 7

To a clear alcoholic (methanol, ethanol, propanol, isopropanol, n-butanol) solution of zofenopril dicyclohexylamine salt, an aqueous solution of calcium chloride dihydrate was added at 50-55°C and the resulting suspension was stirred for 3 hours at 50-55°C (alcohol: water ratio 1:1). Then this suspension was filtered at 25-30°C and the product was washed with water until free from chloride ions. The wet solids were dried under reduced pressure at 55°C until the moisture content fell below 0.5%.
Polymorphic purity >99.9% (as measured by XRPD and DSC)
Chemical purity >99.5% (as measured by HPLC)

### Reference Example 8

To a clear aqueous solution of calcium chloride dihydrate, an alcoholic (methanol, ethanol, propanol, isopropanol, n-butanol) solution of zofenopril dicyclohexylamine salt was added at 25-30°C and the resulting suspension was stirred for 3 hours 25-30°C (alcohol : water ratio 1:1). Then this suspension was filtered at 25-30°C and the product was washed with water until free from chloride ions. The wet solids were dried under reduced pressure at 55°C until the moisture content fell below 0.5%.
Polymorphic purity >99.9% (as measured by XRPD and DSC)
Chemical purity >98.5% (as measured by HPLC)

### Reference Example 9

To a clear aqueous solution of calcium chloride dihydrate, an alcoholic (methanol, ethanol, propanol, isopropanol, n-butanol) solution of zofenopril dicyclohexylamine salt was added at 25-30°C and the resulting suspension was stirred for 3 hours 25-30°C (alcohol : water ratio 1:3). Then this suspension was filtered at 25-30°C and the product was washed with water until free from chloride ions. The wet solids were dried under reduced pressure at 55°C until the moisture content fell below 0.5%.
Polymorphic purity >99.9% (as measured by XRPD and DSC)
Chemical purity >98.5% (as measured by HPLC)

### Reference Example 10

To a clear aqueous solution of calcium chloride dihydrate, an alcoholic (methanol, ethanol, propanol, isopropanol, n-butanol) solution of zofenopril dicyclohexylamine salt was added at 50-55°C and the resulting suspension was stirred for 3 hours at 50-55°C (alcohol : water ratio 1:1). Then this suspension was filtered at 50-55°C and the product was washed with water until free from chloride ions. The wet solids were dried under reduced pressure at 55°C until the moisture content fell below 0.5%.
Polymorphic purity >99.9% (as measured by XRPD and DSC)
Chemical purity >98.5% (as measured by HPLC)

The same crystalline form F of zofenopril calcium was obtained using cyclic ethers (tetrahydrofuran, dioxane) and amides (N,N-dimethylformamide, formamide, N,N-dimethylacetamide) as solvent.

### Example 11: Stability studies of polymorph forms E and F towards possible mechanical stress induced during dosage form preparation

The following studies illustrate the stability of the polymorphic forms according to the present invention to the mechanical stress that occurs during dosage formation by wet granulation.
- Polymorph E when subjected to mechanical stress during particle size reduction (milling using a multimill) retained its polymorphic composition as depicted by thermal data (XRPD, DSC and TGA). This showed that the form E is stable to mechanical stress to which the API would be subjected during dosage form (tablet) preparation.
- The stability of polymorph E was further studied by preparing its slurry in water (10% w/v) and exposing this to 50-55°C for six hours. Thermal data (XRPD, DSC and TGA) recorded after this exposure indicated no change in polymorph E. These results suggested that the polymorph E should be stable to wet granulation.
- In another stress study, a pellet of polymorph E was prepared using an Infrared spectrophotometer press machine (pressure exerted ∼10 Tons). The thermal data (XRPD, DSC and TGA) of this pellet was identical to that before compression. This study also showed the stability of form E towards mechanical stress.

Similar results were seen when stress studies were conducted on form F.
It will be understood that the present invention has been described above by way of example only. The examples are not intended to limit the scope of the invention. Various modifications and embodiments can be made without departing from the scope of the invention, which is defined by the following claims only.

## Claims

1. A process for preparing a polymorphic form of zofenopril calcium, comprising the steps of:
(a) dissolving a salt of zofenopril in a water miscible organic solvent;
(b) mixing the solution obtained in step (a) with an aqueous solution of a calcium salt; and
(c) isolating the polymorphic form of zofenopril calcium;
wherein the polymorphic form is **characterised by** an XRPD spectrum comprising at least six of the following 2θ peaks: 4.2, 4.8, 9.6, 17.5, 18.0, 19.3, 19.9, 20.6 and 24.4 ± 0.2 degrees 2θ;
with the proviso that the salt of zofenopril in step (a) is not an amine salt and wherein steps (a) and (b) are carried out at a temperature of up to 30°C

2. A process according to claim 1, wherein the polymorphic form is **characterised by** a differential scanning calorimetry (DSC) with an endothermic peak at about 255°C.

3. A process according to claim 1 or 2, wherein the polymorphic form is **characterised by**:
(i) an XRPD spectrum substantially as shown in Figure 1; and/or
(ii) a DSC trace substantially as shown in Figure 2; and/or
(iii) a TGA trace substantially as shown in Figure 3.

4. A process according to any one of claims 1 to 3, wherein the polymorphic form comprises less than 10%, less than 5%, less than 1%, less than 0.5%, or less than 0.1% of zofenopril calcium in other polymorphic or amorphous forms.

5. A process according to any one of claims 1 to 4, wherein the water miscible organic solvent is an alcohol, an ether, a cyclic ether, a ketone or an amide.

6. A process according to claim 5, wherein the water miscible organic solvent is:
(i) an alcohol; and/or
(ii) a straight-chain, branched or cyclic C₁ to C₆ alcohol; and/or
(iii) selected from methanol, ethanol, propanol, isopropanol, n-butanol or mixtures thereof.

7. A process according to claim 5, wherein the water miscible organic solvent is:
(i) a cyclic ether; and/or
(ii) selected from tetrahydrofuran and dioxane or mixtures thereof.

8. A process according to claim 5, wherein the water miscible organic solvent is:
(i) an amide; and/or
(ii) selected from N,N-dimethylformamide, formamide and N,N-dimethylacetamide or mixtures thereof.

9. A process according to any one of claims 1 to 8, wherein the zofenopril salt in step (a) is a metal salt.

10. A process according to claim 9, wherein the metal salt is a potassium, sodium, lithium, calcium, magnesium or aluminium salt.

11. A process according to claim 10, wherein the metal salt is a potassium salt.

12. A process according to any one of claims 1 to 11, wherein the calcium salt in step (b) is selected from the fluoride, chloride, bromide, iodide, oxide, hydroxide, carbonate, nitrate, sulfate or acetate salts.

13. A process according to claim 12, wherein the calcium salt is calcium chloride.

14. A process according to any one of claims 1 to 3, wherein the process is carried out at a temperature of from 20 to 30°C.

## Patentansprüche

1. Verfahren zur Herstellung einer polymorphen Form von Zofenopril-Calcium, umfassend die folgenden Schritte:
(a) Auflösen eines Salzes von Zofenopril in einem mit Wasser mischbaren organischen Lösungsmittel;
(b) Mischen der in Schritt (a) erhaltenen Lösung mit einer wässrigen Lösung eines Calciumsalzes; und
(c) Isolieren der polymorphen Form von Zofenopril-Calcium;
wobei die polymorphe Form durch ein XRPD-Spektrum gekennzeichnet ist, das mindestens sechs der folgenden 20 Spitzenwerte umfasst: 4,2, 4,8, 9,6, 17,5, 18, 0, 19,3, 19,9, 20,6 und 24,4 ± 0,2 Grad 20;
mit der Maßgabe, dass das Salz von Zofenopril in Schritt (a) kein Aminsalz ist und wobei die Schritte (a) und (b) bei einer Temperatur von bis zu 30 °C durchgeführt werden.

2. Verfahren nach Anspruch 1, wobei die polymorphe Form durch eine Differential-Scanning-Calorimetrie (DSC) mit einem endothermen Spitzenwert bei etwa 255 °C gekennzeichnet ist.

3. Verfahren nach Anspruch 1 oder 2, wobei die polymorphe Form **gekennzeichnet ist durch**:
(i) ein XRPD-Spektrum im Wesentlichen wie in Figur 1 dargestellt; und/oder
(ii) eine DSC-Spur im Wesentlichen wie in Figur 2 dargestellt; und/oder
(iii) eine TGA-Spur im Wesentlichen wie in Figur 3 dargestellt.

4. Verfahren nach einem der Ansprüche 1 bis 3, wobei die polymorphe Form weniger als 10 %, weniger als 5 %, weniger als 1 %, weniger als 0,5 % oder weniger als 0,1 % Zofenopril-Calcium in anderen polymorphen oder amorphen Formen umfasst.

5. Verfahren nach einem der Ansprüche 1 bis 4, wobei das mit Wasser mischbare organische Lösungsmittel ein Alkohol, ein Ether, ein cyclischer Ether, ein Keton oder ein Amid ist.

6. Verfahren nach Anspruch 5, wobei das mit Wasser mischbare organische Lösungsmittel Folgendes ist:
(i) ein Alkohol; und/oder
(ii) ein geradkettiges, verzweigtes oder zyklisches C₁ bis C₆ Alkohol; und/oder
(iii) ausgewählt aus Methanol, Ethanol, Propanol, Isopropanol, n-Butanol oder Gemischen davon.

7. Verfahren nach Anspruch 5, wobei das mit Wasser mischbare organische Lösungsmittel Folgendes ist:
(i) ein zyklisches Ether; und/oder
(ii) ausgewählt aus Tetrahydrofuran und Dioxan oder Gemischen davon.

8. Verfahren nach Anspruch 5, wobei das mit Wasser mischbare organische Lösungsmittel Folgendes ist:
(i) Amid; und/oder
(ii) ausgewählt aus N,N-Dimethylformamid, Formamid und N,N-Dimethylacetamid oder Gemischen davon.

9. Verfahren nach einem der Ansprüche 1 bis 8, wobei das Zofenopril-Salz in Schritt (a) ein Metallsalz ist.

10. Verfahren nach Anspruch 9, wobei das Metallsalz ein Kalium-, Natrium-, Lithium-, Calcium-, Magnesium- oder Aluminiumsalz ist.

11. Verfahren nach Anspruch 10, wobei das Metallsalz ein Kaliumsalz ist.

12. Verfahren nach einem der Ansprüche 1 bis 11, wobei das Calciumsalz in Schritt (b) aus Fluorid, Chlorid, Bromid, Iodide, Oxid, Hydroxid, Carbonat, Nitrat, Sulfat oder Acetatsalzen ausgewählt wird.

13. Verfahren nach Anspruch 12, wobei das Calciumsalz Calciumchlorid ist.

14. Verfahren nach einem der Ansprüche 1 bis 3, wobei das Verfahren bei einer Temperatur von 20 bis 30 °C durchgeführt wird.

## Revendications

1. Procédé de préparation d'une forme polymorphique de zofénopril calcium, comprenant les étapes de :
(a) la dissolution d'un sel de zofénopril dans un solvant organique miscible à l'eau ;
(b) le mélange de la solution obtenue à l'étape (a) avec une solution aqueuse d'un sel de calcium ; et
(c) l'isolation de la forme polymorphique de zofénopril calcium ;
dans lequel le forme polymorphique se **caractérise par** un spectre XRPD comprenant au moins six des 20 pics suivants : 4,2, 4,8, 9,6, 17,5,18,0, 19,3, 19,9, 20,6 et 24,4 ± 0,2 degrés 20 ;
à condition que le sel de zofénopril à l'étape (a) ne soit pas un sel d'amine et dans lequel les étapes (a) et (b) sont effectuées à une température allant jusqu'à 30°C

2. Procédé selon la revendication 1, dans lequel la forme polymorphe est **caractérisée par** une calorimétrie différentielle à balayage (DSC) avec un pic endothermique à environ 255°C.

3. Procédé selon la revendication 1 ou 2, dans lequel la forme polymorphique est **caractérisée par** :
(i) un spectre XRPD sensiblement tel que montré à la Figure 1 ; et/ ou
(ii) une trace DSC sensiblement tel que montrée à la Figure 2 ; et/ ou
(iii) une trace de TGA, sensiblement tel que montrée à la Figure 3.

4. Procédé selon l'une quelconque des revendications 1 à 3, dans lequel la forme polymorphique comprend moins de 10%, moins de 5%, moins de 1%, moins de 0,5%, ou moins de 0,1% de zofénopril calcium dans d'autres formes polymorphiques ou amorphes.

5. Procédé selon l'une quelconque des revendications 1 à 4, dans lequel le solvant organique miscible à l'eau est un alcool, un éther, un éther cyclique, une cétone ou un amide.

6. Procédé selon la revendication 5, dans lequel le solvant organique miscible à l'eau est :
(i) un alcool ; et/ ou
(ii) un alcool en C₁ à C₆ à chaîne droite, ramifiée ou cyclique ; et/ ou
(iii) sélectionnés parmi le méthanol, l'éthanol, le propanol, l'isopropanol, le n-butanol ou les mélanges de ceux-ci.

7. Procédé selon la revendication 5, dans lequel le solvant organique miscible à l'eau est :
(i) une éther cyclique ; et/ ou
(ii) sélectionné parmi le tétrahydrofurane et le dioxane ou leurs mélanges.

8. Procédé selon la revendication 5, dans lequel le solvant organique miscible à l'eau est :
(i) un amide ; et/ ou
(ii) sélectionné parmi le N, N-diméthylformamide, le formamide et le N, N-diméthylacétamide ou leurs mélanges.

9. Procédé selon l'une quelconque des revendications 1 à 8, dans lequel le sel de zofénopril à l'étape (a) est un sel métallique.

10. Procédé selon la revendication 9, dans lequel le sel métallique est un sel de potassium, de sodium, de lithium, de calcium, de magnésium ou d'aluminium.

11. Procédé selon la revendication 10, dans lequel le sel métallique est un sel de potassium.

12. Procédé selon l'une quelconque des revendications 1 à 11, dans lequel le sel de calcium à l'étape (b) est sélectionné parmi les sels de fluorure, de chlorure, de bromure, d'iodure, d'oxyde, d'hydroxyde, de carbonate, de nitrate, de sulfate ou d'acétate.

13. Procédé selon la revendication 12, dans lequel le sel de calcium est le chlorure de calcium.

14. Procédé selon l'une quelconque des revendications 1 à 3, dans lequel le procédé est effectué à une température de 20 à 30°C.
